# EUROPÄISCHE PATENTSCHRIFT

(11) **EP 0 755 933 B1**
(45) Veröffentlichungstag und Bekanntmachung des Hinweises auf die Patenterteilung: **27.03.2002**
(21) Anmeldenummer: 96810391.1
(22) Anmeldetag: 13.06.1996
(51) Int. Cl.: C07D 487/04, C08K 5/34, C07F 9/6561, C09B 57/00

(54) **Neue blaue Diketopyrrolopyrrolpigmente**
Blue diketopyrrolopyrrole pigments
Pigments bleu du type dicétopyrrolopyrrole

(30) Priorität: 22.06.1995 CH 183695
(43) Veröffentlichungstag der Anmeldung: 29.01.1997
(73) Patentinhaber: Ciba Specialty Chemicals Holding Inc., 4057 Basel (CH)
(72) Erfinder: Wallquist, Olof, Dr., 1723 Marly (CH); Lamatsch, Bernd, Dr., 1723 Marly (CH); Ruch, Thomas, Dr., 1723 Marly (CH)

(56) Entgegenhaltungen:
- EP-A- 0 511 165
- CHEMICAL ABSTRACTS, vol. 113, no. 8, 1990 Columbus, Ohio, US; abstract no. 68369j, T. TOSHIO ET AL.: "Electrophotographic photoconductors" Seite 68365; XP002014666 & JP-A-00 255 362 (TOYO) 23.Februar 1990

## Beschreibung

Die vorliegende Erfindung betrifft neue, beständige blau bis violette Diketopyrrolopyrrole und ihre Verwendung als Pigmente.

1,4-Diketopyrrolo[3,4-c]pyrrole sind seit einigen Jahren als Pigmente mit ausgezeichneten Pigmenteigenschaften bekannt, so z.B. aus US-Patent 4 415 685, wo Diketopyrrolopyrrole der Formel 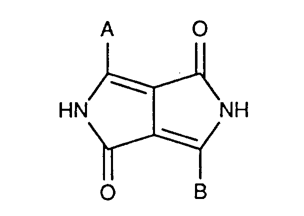 worin A und B isocyclische oder heterocyclische Reste, bevorzugt mono- bis tetracyclische, insbesondere mono- oder bicyclische Reste bedeuten, als rote Pigmente mit hoher Farbtonreinheit, hoher Farbstärke und guten Beständigkeiten, wie z.B. Licht-, Wetter-, Hitze- und Migrationsbeständigkeit, beschrieben sind. Die gute Eignung solcher Produkte als Pigmente mit oranger bis insbesondere roter Nuance wird auch in zahlreichen Folgepatenten, wie z.B. in US 4 579 949, US 4 720 305, US 4 810 802, US 4 783 540, US 5 200 528, u.a.m., bestätigt. In US-Patent 4 579 949 wird auch ein Diketopyrrolopyrrol der obenerwähnten Formel, worin A und B p-Dimethylaminophenyl bedeuten, als blaues Pigment beschrieben. Dieses genügt allerdings bezüglich Licht- und Wetterbeständigkeit nicht den heutigen Anforderungen der Technik.

Es ist nun gefunden worden, dass Diketopyrrolopyrrole der obenerwähnten Formel, worin A und B zwei verschiedene, substituierte aromatische Reste, wovon einer eine Aminogruppe enthält, bedeuten, ganz überraschend blau bis violette Pigmente mit verbesserten Pigmenteigenschaften insbesondere Licht- und Wetterbeständigkeit sind.

Die vorliegende Erfindung betrifft demnach Diketopyrrolo[3,4-c]pyrrole der Formel 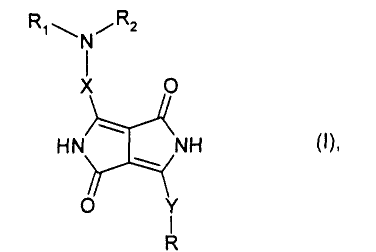 worin X und Y unabhängig voneinander einen zweiwertigen aromatischen Rest der Formel 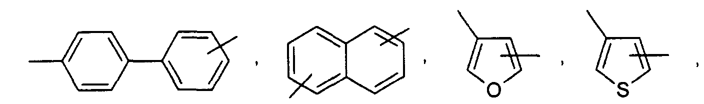 oder insbesondere 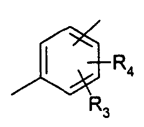 bedeuten,
R ein Rest CN, COR₅, CO₂R₅, CON(R₅)₂, NO₂, SO₂R₅, SOR₅, SO₂N(R₅)₂ oder PO(OR₆)₂ ist,
R₃ und R₄ unabhängig voneinander Wasserstoff, Chlor, Brom, Methyl, Ethyl, Methoxy oder Ethoxy bedeuten,
R₆ C₁-C₆-Alkyl oder Phenyl ist,
R₁, R₂ und R₅ unabhängig voneinander Wasserstoff, C₁-C₁₈-Alkyl, C₂-C₁₈-Alkenyl, unsubstituiertes oder durch Chlor, Brom, Hydroxy, C₁-C₆-Alkyl, C₁-C₆-Alkoxy, C₁-C₆-Alkylmercapto, CN, NO₂ oder CF₃ substituiertes Phenyl bedeuten,
oder R₁ und R₂ zusammen mit dem N-Atom, an das sie gebunden sind, einen unsubstituierten oder durch C₁-C₆-Alkyl oder Phenyl substituierten heterocyclischen Rest ausgewählt aus der Gruppe Pyrrolidinyl, Piperidyl, Pyrrolyl, Triazolyl, Imidazolyl, Pyrazolyl, Piperazinyl, Morpholinyl, Thiomorpholinyl, Carbazol-1-yl, Indol-1-yl, Indazol-1-yl, Benzimidazol-1-yl, Tetrahydrochinolin-1-yl und Tetrahydrochinolin-2-yl bilden oder,
wenn R₁ Wasserstoff ist, R₂ einen Rest der Formel 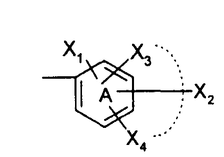
bedeutet, worin X₁ und X₂ unabhängig voneinander Wasserstoff, Chlor, Brom, NO₂, Methyl, Methoxy oder Ethoxy sind und X₃ und X₄ einen heterocyclischen Ring bilden, der zusammen mit A einen Benzimidazolonyl-, Dihydroxychinazolinyl-, Chinolonyl-, Benzoxazolonyl-, Phenmorpholonyl-, Chinazolinonyl-, Phthalimidylrest oder einen Rest der Formel 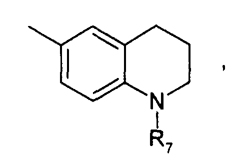 worin R₇ C₁-C₆-Alkyl oder Phenyl ist,
ergibt oder
Y-R ein Rest 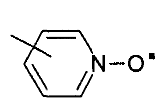 sein kann.

C₁-C₆-Alkyl steht z.B. für Methyl, Ethyl, n-Propyl, Isopropyl, n-Butyl, sec-Butyl, tert.-Butyl, Amyl, Hexyl und C₁-C₁₈-Alkyl zusätzlich für z.B. Heptyl, Octyl, 2-Ethylhexyl, Nonyl, Decyl, Dodecyl, Tetradecyl, Hexadecyl oder Octadecyl. Die gleiche Bedeutung kann C₁-C₆-Alkyl auch in C₁-C₆-Alkylmercapto haben.

C₂-C₁₈-Alkenyl bedeutet z.B. Vinyl, Allyl, Methallyl, n-But-2-enyl, 2-Methyl-prop-2-enyl, n-Pent-2-enyl, n-Hex-2-enyl, n-Oct-2-enyl, n-Dec-2-enyl, n-Dodec-2-enyl oder Octadec-5-enyl.

Bei C₁-C₆-Alkoxy handelt es sich z.B. um Methoxy, Ethoxy, n-Propoxy, Isopropoxy, n-Butoxy, tert.-Butoxy, n-Pentoxy, tert.-Amyloxy oder Hexyloxy.

Der lange Valenzstrich bei X₂ bedeutet, dass X₂ entweder im aromatischen Ring A oder

Die Reste der Formel 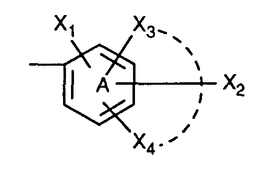 für R₂ leiten sich von Aminen der Formel 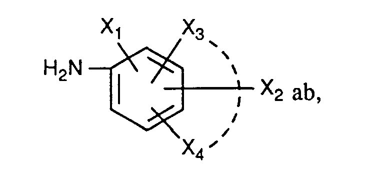 wobei X₁ - X₄ die oben angegebene Bedeutung haben.

### Beispiele hierfür sind:

5-Amino-benzimidazolon, 7-Chlor-5-amino-benzimidazolon, 7-Brom-5-amino-benzimidazolon, 6-Chlor-5-aminobenzimidazolon, 6-Brom-5-amino-benzimidazolon, 6-Methoxy-5-amino-benzimidazolon, 7-Methoxy-5-amino-benzimidazolon, 6-Ethoxy-5-amino-benzimidazolon, 7-Chlor-4-methyl-5-amino-benzimidazolon, 6-Methyl-5-aminobenzimidazolon, 4,7-Dimethyl-5-amino-benzimidazolon, 4-Methyl-6-chlor-5-amino-benzimidazolon, 5-Amino-1-methyl-benzimidazolon, 6-Amino-2,4-dihydroxychinazolin, 6-Amino-1,4-dihydroxychinazolin, 6-Amino-4-methyl-chinolon-2, 7-Amino-4-methylchinolon-2, 7-Amino-4,6-dimethyl-chinolon-2, 6-Amino-7-chlor-4-methyl-chinolon-2, 7-Amino-4-methyl-6-methoxy-chinolon-2, 5-Aminobenzoxazolon, 6-Aminobenzoxazolon, 6-Amino-5-methyl-benzoxazolon, 6-Amino-5-chlor-benzoxazolon, 6-Aminophenmorpholon-3, 7-Amino-phenmorpholon-3, 7-Amino-6-chlor-phenmorpholon-3, 7-Amino-6-methyl-phenmorpholon-3, 7-Amino-6-methoxy-phenmorpholon-3, 6-Amino-4-methyl-phenmorpholon-3, 7-Amino-4-methyl-phenmorpholon-3, 7-Amino-4,6-dimethyl-phenmorpholon-3, 6-Amino-chinazolinon-4 und 5-Aminophthalimid.

Von besonderem Interesse sind Diketopyrrolo[3,4-c]pyrrole der Formel I, worin X und Y unabhängig voneinander 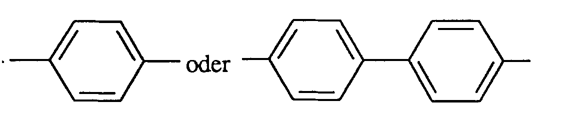 bedeuten,
R ein Rest CN, NO₂, CO₂R₅ oder CON(R₅)₂ ist,
R₁, R₂ und R₅ unabhängig voneinander Wasserstoff, C₁-C₆-Alkyl, unsubstituiertes oder durch Chlor, Brom, C₁-C₅-Alkyl, Methoxy, CN oder NO₂ substituiertes Phenyl bedeuten oder R₁ und R₂ zusammen mit dem N-Atom an das sie gebunden sind Pyrrolidinyl oder Piperidin bilden.

Bevorzugt sind Diketopyrrolo[3,4-c]pyrrole der Formel I, worin X und Y 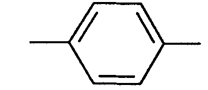 bedeuten,
R ein Rest CN oder NO₂ ist,
R₁ und R₂ unabhängig voneinander Wasserstoff, Methyl, unsubstituiertes oder durch Methyl, Methoxy, Ethoxy, Cl oder NO₂ substituiertes Phenyl oder R₁ und R₂ zusammen mit dem N-Atom an das sie gebunden sind Pyrrolidinyl oder Piperidyl bilden.
R₁ und R₂ sind vorzugsweise gleich.

Die erfindungsgemässen Diketopyrrolo[3,4-c]pyrrole der Formel I können in Analogie zu allgemein bekannten Verfahren hergestellt werden, z.B. durch Umsetzung eines Diketopyrrolo[3,4-c]pyrrols der Formel 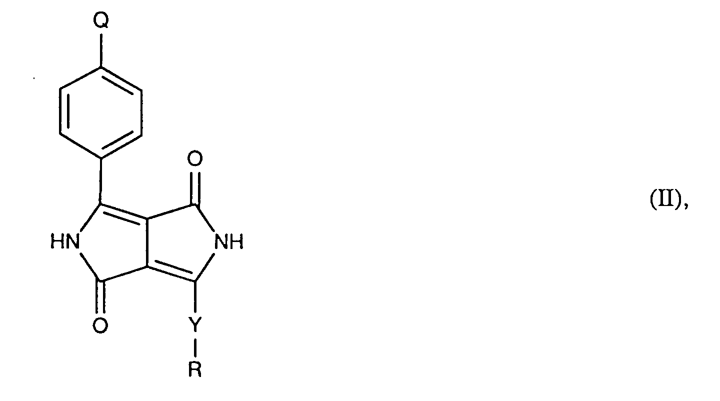 worin Q Brom oder bevorzugt Fluor und insbesondere Chlor bedeutet und Y und R die oben angegebene Bedeutung haben,
mit einem Amin der Formel 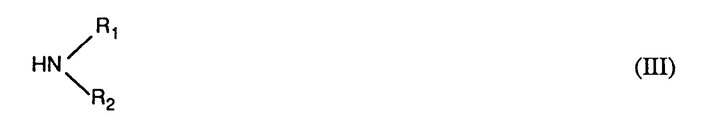 wie dies beispielsweise in EP-A 353 184 beschrieben ist.

Die Diketopyrrolo[3,4-c]pyrrole der Formel II sind bekannte Substanzen. Sollten einige noch neu sein, so können sie in Analogie zu allgemein bekannten Verfahren, z.B. durch Umsetzung eines Pyrrolinons der Formel 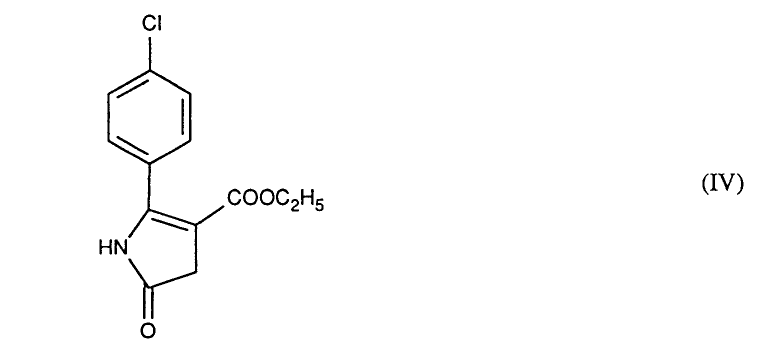 mit einem Nitril der Formel R-Y-CN (V) wie z.B. in US-Patent 4 659 775 beschrieben, hergestellt werden.

Die Verbindungen der Formeln III, IV und V sind bekannte Substanzen. Sollten einige noch neu sein, so können sie nach allgemein bekannten Verfahren hergestellt werden.

Die erfindungsgemässen Diketopyrrolo[3,4-c]pyrrole können als Pigmente zum Färben von hochmolekularem organischem Material verwendet werden.

Hochmolekulare organische Materialien, die mit den erfindungsgemässen Diketopyrrolo-[3,4-c]pyrrolen pigmentiert werden können, sind z.B. Celluloseether und -ester, wie Ethylcellulose, Nitrocellulose, Celluloseacetat, Cellulosebutyrat, natürliche Harze oder Kunstharze, wie Polymerisationsharze oder Kondensationsharze, wie Aminoplaste, insbesondere Harnstoff- und Melamin-Formaldehydharze, Alkydharze, Phenoplaste, Polycarbonate, Polyolefine, Polystyrol, Polyvinylchlorid, Polytetrafluoroethylen, Polyamide, Polyurethane, Polyester, Polyether-ketone, Polyphenylenoxide, Gummi, Casein, Silikon und Silikonharze, einzeln oder in Mischungen.

Die erwähnten hochmolekularen organischen Verbindungen können einzeln oder in Gemischen als plastische Massen, Schmelzen oder in Form von Spinnlösungen, Lacken, Anstrichstoffen oder Druckfarben vorliegen. Je nach Verwendungszweck erweist es sich als vorteilhaft, die erfindungsgemässen Diketopyrrolo[3,4-c]pyrrole als Toner oder in Form von Präparaten einzusetzen. Bezogen auf das zu pigmentierende hochmolekulare organische Material kann man die erfindungsgemässen Diketopyrrolo[3,4-c]pyrrole in einer Menge von 0,01 bis 30 Gew.%, vorzugsweise von 0,1 bis 10 Gew.%, einsetzen.

Zum Pigmentieren von Lacken und Druckfarben werden die hochmolekularen organischen Materialien und die erfindungsgemässen Diketopyrrolo[3,4-c]pyrrole gegebenenfalls zusammen mit Zusatzstoffen, wie Füllmitteln, anderen Pigmenten, Siccativen oder Weichmachern, in einem gemeinsamen organischen Lösungsmittel oder Lösungsmittelgemisch fein dispergiert bzw. gelöst. Man kann dabei so verfahren, dass man die einzelnen Komponenten für sich oder auch mehrere gemeinsam dispergiert bzw. löst, und erst hierauf alle Komponenten zusammenbringt.

Die erhaltenen blau bis violetten Färbungen, beispielsweise in Kunststoffen, Fasern, Lacken oder Drucken, zeichnen sich durch gute allgemeine Eigenschaften, wie hohe Farbstärke, gute Dispergierbarkeit, Ueberlackier-, Migrations-, Hitze-, und insbesondere durch gute Licht- und Wetterbeständigkeit aus.

Die nachfolgenden Beispiele erläutern die Erfindung.
Beispiel 1: In einem Autoklaven werden 5,0 g 3-(4-Chlorphenyl)-6-(4-cyanophenyl)-2,5-dihydropyrrolo[3,4-c]pyrrol-1,4-dion (bisher in der Patentliteratur als 1,4-Diketo-3-(4-chlorphenyl)-6-(4-cyanophenyl)-pyrrolo[3,4-c]pyrrol bezeichnet) in 100 ml N-Methylpyrrolidon suspendiert. 10,7 g Dimethylamingas werden dann zugeführt und das Gemisch 10 Stunden bei 180°C gerührt. Anschliessend wird das Gemisch mit N-Methylpyrrolidon herausgespült, filtriert, mit Methanol und Wasser bis farblos gewaschen und im Vakuumtrockenschrank bei 80°C getrocknet. Man erhält 3,6 g eines blauvioletten Pulvers.

| Analyse (C₂₁H₁₆N₄O₂) | C | H | N |
|---|---|---|---|
| Ber. | 70,8 % | 4,5% | 15,7 % |
| Gef. | 69,8 % | 4,5 % | 15,4 % |

Beispiel 2: Analog zu Beispiel 1 werden 3,8 g 3-(4-Chlorphenyl)-6-(4-cyanophenyl)-2,5-dihydropyrrolo[3,4-c]pyrrol-1,4-dion mit 13,8 g Pyrrolidin in 80 ml N-Methylpyrrolidon umgesetzt. Man erhält 0,45 g eines blauvioletten Pulvers.

| Analyse (C₂₃H₁₈N₄O₂) | C | H | N |
|---|---|---|---|
| Ber. | 72,2 % | 4,7 % | 14,6 % |
| Gef. | 70,9 % | 4,7 % | 14,5 % |

Beispiel 3: Analog zu Beispiel 1 werden 10,0 g 3-(4-Chlorphenyl)-6-(4-dimethylaminocarbonyl-phenyl)-2,5-dihydropyrrolo[3,4-c]pyrrol-1,4-dion mit 18,7 g Dimethylamin in 200 ml N-Methylpyrrolidon umgesetzt. Man erhält 0,3 g eines blauvioletten Pulvers, in dem zu ca. 44 % (Cl-Analyse) das Chlor im Edukt gegen Dimethylamino substituiert wurde.

| Analyse (C₂₃H₂₈N₄O₃) | C | H | N | Cl |
|---|---|---|---|---|
| Ber. | 68,6 % | 5,5 % | 13,9 % | 0 % |
| Gef. | 60,2 % | 3,9% | 9,0 % | 5,0 % |

Beispiel 4:1,0 g des in Beispiel 1 erhaltenen Produktes wird in 10 ml konzentrierter Schwefelsäure bei 0°C gelöst und während 16 Stunden unter Stickstoff gerührt, wobei man das Reaktionsgemisch langsam auf Raumtemperatur zurückkommen lässt. Das Reaktionsgemisch wird anschliessend auf ein Eis-Wasser Gemisch gegossen und mit 30 % Natronlauge neutralisiert. Das Pigment wird abgenutscht, mit 100 ml Wasser nachgewaschen und im Trockenschrank über Nacht getrocknet. Man erhält 0,95 g eines blauvioletten Pulvers.

| Analyse (C₂₁H₁₈N₄O₃) | C | H | N |
|---|---|---|---|
| Ber. | 67,4% | 4,9 % | 15,0 % |
| Gef. | 65,8 % | 5,2 % | 14,6 % |

im IR kein CN-Signal mehr.
Beispiel 5: Eine Lösung von 32,4 g 3-(4-Chlorphenyl)-6-phenyl-2,5-dihydropyrrolo[3,4-c]pyrrol-1,4-dion in 740 g wasserfreier Schwefelsäure wird auf 0°C abgekühlt und bei dieser Temperatur im Laufe einer Stunde mit 10,6 g Kaliumnitrat versetzt. Nach weiteren fünf Stunden Rühren, ohne die Temperatur von +5°C zu überschreiten, wird die entstandene Lösung stets unter Rühren mit der Zahnscheibe auf 2000 Teile Eis ausgetragen. Nach Abfiltrieren der erhaltenen Suspension, Neutralwaschen mit Wasser und Trocknen bei 80°C im Vakuum erhält man 19,4 g eines violetten Pigments.

| Analyse (C₁₈H₁₀ClN₃O₄) | C | H | N | Cl |
|---|---|---|---|---|
| Ber. | 58,8 % | 2,7 % | 11,4 % | 9,6 % |
| Gef. | 58,0 % | 2,9 % | 11,0 % | 9,6 % |

Beispiel 6: In 100 Teilen N-Methylpyrrolidon werden 3,67 g des in Beispiel 5 erhaltenen Pigmentes suspendiert und im Autoklaven 10 g Dimethylamin aufgepresst. Nach 10-stündigem Heizen auf 180°C unter Rühren wird die erhaltene Suspension auf Raumtemperatur abgekühlt und stets unter Rühren mit der Zahnscheibe auf 1000 g Wasser ausgetragen. Nach gründlichem Waschen mit Wasser und Trocknen bei 80°C im Vakuum erhält man 1,6 g eines violetten Pigmentes.

| Analyse (C₂₀H₁₆N₄O₄) | C | H | N | Cl |
|---|---|---|---|---|
| Ber. | 63,8 % | 4,3 % | 14,9 % | 0,0 % |
| Gef. | 66,8 % | 5,2 % | 14,4 % | 2,2 % |

Beispiel 7: Analog zu Beispiel 1 werden 4,3 g 3-(4-Chlorphenyl)-6-(6-cyanonaphthalin-2-yl)-2,5-dihydropyrrolo[3,4-c]pyrrol-1,4-dion mit 9,0 g Dimethylamin in 100 ml N-Methylpyrrolidon umsetzt. Man erhält 3,7 g eines blauvioletten Pulvers.

| Analyse (C₂₅H₁₈N₄O₂) | C | H | N | Cl |
|---|---|---|---|---|
| Ber. | 73,9 % | 4,5 % | 13,8 % | 0 % |
| Gef. | 72,6 % | 4,3 % | 13,0 % | 1,9 % |

Beispiel 8: Analog zu Beispiel 1 werden 5,2 g 3-(4-Chlorphenyl)-6-(4-cyanobiphenyl)-2,5-dihydropyrrolo[3,4-c]pyrrol-1,4-dion mit 10,9 g Dimethylamin in 100 ml N-Methylpyrrolidin umgesetzt Man erhält 2,8 g eines blauvioletten Pulvers.

| Analyse (C₂₇H₂₀N₄O₂) | C | H | N | Cl |
|---|---|---|---|---|
| Ber. | 74,99 % | 4,66 % | 12,95 % | 0 % |
| Gef. | 73,9 % | 4,6 % | 12,6 % | 0,9 % |

Beispiel 9: 7,5 g des Pigments aus Beispiel 1, 98,9 g CAB-Lösung bestehend aus

| | |
|---|---|
| 41,0 g | Celluloseacetobutyrat ® CAB 531.1, 20 %ig in Butanol/Xylol 2:1 (Eastman Chem.) |
| 1,5 g | Zirkonium Octoat, |
| 18,5 g | ® SOLVESSO 150* (ESSO), |
| 21,5 g | Butylacetat und |
| 17,5 g | Xylol, |

| | |
|---|---|
| * Aromatische Kohlenwasserstoffe | |

36,5 g Polyesterharz ® DYNAPOL H700 (Dynamit Nobel), 4,6 g Melaminharz MAPRENAL MF650 (Hoechst) und 2,5 g Dispergiermitel ® DISPERBYK 160 (Byk Chemie) werden zusammen während 90 Minuten mit einer Schüttelmaschine dispergiert (Total Lack 150 g; 5 % Pigment).
27,69 g des so erhaltenen Volltonlacks werden für die Base-coat-Lackierung mit 17,31 g Al-Stammlösung (8 %ig) bestehend aus

| | |
|---|---|
| 12,65 g | ® SILBERLINE SS 3334AR, 60 %ig (Silberline Ltd.) |
| 56,33 g | CAB Lösung (Zusammensetzung wie oben) |
| 20,81 g | Polyesterharz ® DYNAPOL H700 |
| 2,60 g | Melaminharz ® MAPRENAL MF650 |
| 7,59 g | ® SOLVESSO 150 |

gemischt und auf ein Aluminiumblech spritzappliziert (Nassfilm ca. 20 µm). Nach einer Abdunstzeit von 30 Minuten bei Raumtemperatur wird ein TSA-Lack bestehend aus

| | |
|---|---|
| 29,60 g | Acrylharz ® URACRON 2263 XB, 50 %ig in Xylol/Butanol (Chem. Fabrik Schweizerhalle), |
| 5,80 g | Melaminharz ® CYMEL 327, 90 %ig in Isobutanol, |
| 2,75 g | Butylglycolacetat, |
| 5,70 g | Xylol, |
| 1,65 g | n-Butanol |
| 0,50 g | Siliconöl, 1 %ig in Xylol, |
| 3,00 g | Lichtschutzmittel ® TINUVIN 900, 10 %ig in Xylol (Ciba) |
| 1,00 g | Lichtschutzmittel ® TINUVIN 292, 10 %ig in Xylol (Ciba) |

als Top-coat-Lackierung spritzappliziert (Nassfilm ca. 50 µm). Anschliessend wird der Lack nach weiteren 30 Minuten Abdunsten bei Raumtemperatur, 30 Minuten bei 130°C eingebrannt. Man erhält eine blauviolette Lackierung mit sehr guten Beständigkeiten.
Beispiel 10: 0,6 g des Pigments von Beispiel 1 werden mit 67 g Polyvinylchlorid, 33 g Dioctylphthalat, 2 g Dibutylzinndilaurat und 2 g Titandioxid vermischt und auf einem Walzenstuhl während 15 Minuten bei 160°C zu einer dünnen Folie verarbeitet. Die so erzeugte blauviolette PVC-Folie ist sehr farbstark, migrations- und lichtbeständig.
Beispiel 11: 1000 g Polypropylengranulat (® DAPLEN PT-55, Chemie LINZ) und 20 g eines 50 %-igen Pigmentpräparates, bestehend aus 10 g des Pigments von Beispiel 1 und 10 g Mg-Behenat, werden in einer Mischtrommel intensiv vermischt. Das so behandelte Granulat wird bei 260 bis 285°C nach dem Schmelzspinnverfahren versponnen. Man erhält blauviolett gefärbte Fasern mit sehr guten Licht- und textilen Echtheiten.

## Patentansprüche

1. Diketopyrrolo[3,4-c]pyrrole der Formel 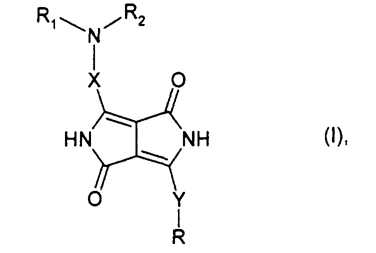 worin X und Y unabhängig voneinander einen zweiwertigen aromatischen Rest der Formel 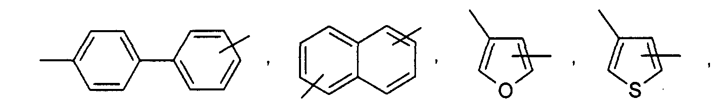 oder 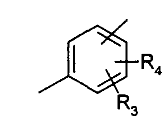 bedeuten,
R ein Rest CN, COR₅, CO₂R₅, CON(R₅)₂, NO₂, SO₂R₅, SOR₅, SO₂N(R₅)₂ oder PO(OR₆)₂ ist,
R₃ und R₄ unabhängig voneinander Wasserstoff, Chlor, Brom, Methyl, Ethyl, Methoxy oder Ethoxy bedeuten,
R₆ C₁-C₆-Alkyl oder Phenyl ist,
R₁, R₂ und R₅ unabhängig voneinander Wasserstoff, C₁-C₁₈-Alkyl, C₂-C₁₈-Alkenyl, unsubstituiertes oder durch Chlor, Brom, Hydroxy, C₁-C₆-Alkyl, C₁-C₆-Alkoxy, C₁-C₆-Alkylmercapto, CN, NO₂ oder CF₃ substituiertes Phenyl bedeuten,
oder R₁ und R₂ zusammen mit dem N-Atom, an das sie gebunden sind, einen unsubstituierten oder durch C₁-C₆-Alkyl oder Phenyl substituierten heterocyclischen Rest ausgewählt aus der Gruppe Pyrrolidinyl, Piperidyl, Pyrrolyl, Triazolyl, Imidazolyl, Pyrazolyl, Piperazinyl, Morpholinyl, Thiomorpholinyl, Carbazol-1-yl, Indol-1-yl, Indazol-1-yl, Benzimidazol-1-yl, Tetrahydrochinolin-1-yl und Tetrahydrochinolin-2-yl bilden oder,
wenn R₁ Wasserstoff ist, R₂ einen Rest der Formel 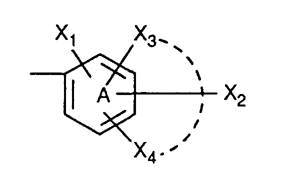
bedeutet, worin X₁ und X₂ unabhängig voneinander Wasserstoff, Chlor, Brom, NO₂, Methyl, Methoxy oder Ethoxy sind und X₃ und X₄ einen heterocyclischen 5- oder 6-Ring bilden, der zusammen mit A einen Benzimidazolonyl-, Dihydroxychinazolinyl-, Chinolonyl-, Benzoxazolonyl-, Phenmorpholonyl-, Chinazolinonyl-, Phthalimidylrest oder einen Rest der Formel 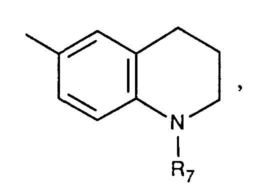 worin R₇ C₁-C₆-Alkyl oder Phenyl ist,
ergibt oder
Y-R ein Rest 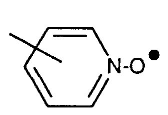 sein kann.

2. Diketopyrrolo[3,4-c]pyrrole gemäss Anspruch 1 der Formel I, worin
X und Y unabhängig voneinander 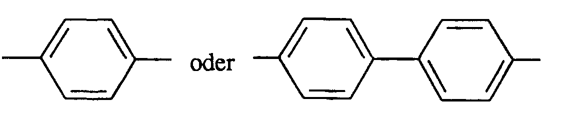 bedeuten,
R ein Rest CN, NO₂, CO₂R₅ oder CON(R₅)₂ ist,
R₁, R₂ und R₅ unabhängig voneinander Wasserstoff, C₁-C₆-Alkyl, unsubstituiertes oder durch Chlor, Brom, C₁-C₅-Alkyl, Methoxy, CN oder NO₂ substituiertes Phenyl bedeuten oder R₁ und R₂ zusammen mit dem N-Atom an das sie gebunden sind Pyrrolidinyl oder Piperidin bilden.

3. Diketopyrrolo[3,4-c]pyrrole gemäss Anspruch 1 der Formel I, worin X und Y 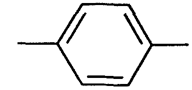 bedeuten
R ein Rest CN oder NO₂ ist,
R₁ und R₂ unabhängig voneinander Wasserstoff, Methyl, unsubstituiertes oder durch Methyl, Methoxy, Ethoxy, Cl oder NO₂ substituiertes Phenyl oder R₁ und R₂ zusammen mit dem N-Atom an das sie gebunden sind Pyrrolidinyl oder Piperidyl bilden.

4. Diketopyrrolo[3,4-c]pyrrole gemäss Anspruch 3 der Formel I, worin R₁ und R₂ gleich sind.

5. Mit einem Diketopyrrolo[3,4-c]pyrrol gemäss Anspruch 1 pigmentiertes hochmolekulares organisches Material.

## Claims

1. A diketopyrrolo[3,4-c]pyrrole of the formula 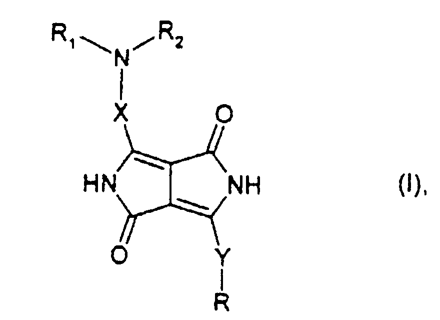 in which X and Y independently of one another are a divalent aromatic radical of the formula 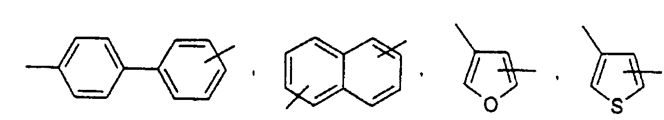 or 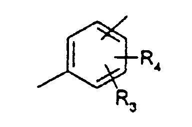
R is a radical CN, COR₅, CO₂R₅, CON(R₅)₂, NO₂, SO₂R₅, SOR₅, SO₂N(R₅)₂ or PO(OR₆)₂,
R₃ and R₄ independently of one another are hydrogen, chlorine, bromine, methyl, ethyl, methoxy or ethoxy,
R₆ is C₁-C₆alkyl or phenyl,
R₁, R₂ and R₅ independently of one another are hydrogen, C₁-C₁₈alkyl, C₂-C₁₈alkenyl, or phenyl which is unsubstituted or substituted by chlorine, bromine, hydroxyl, C₁-C₆alkyl, C₁-C₆alkoxy, C₁-C₆alkylmercapto, CN, NO₂ or CF₃,
or R₁ and R₂, together with the nitrogen atom to which they are attached, form a heterocyclic radical which is unsubstituted or substituted by C₁-C₆alkyl or phenyl and is selected from the group consisting of pyrrolidinyl, piperidyl, pyrrolyl, triazolyl, imidazolyl, pyrazolyl, piperazinyl, morpholinyl, thiomorpholinyl, carbazol-1-yl, indol-1-yl, indazol-1-yl, benzimidazol-1-yl, tetrahydroquinol-1-yl and tetrahydroquinol-2-yl, or,
if R₁ is hydrogen, R₂ is a radical of the formula 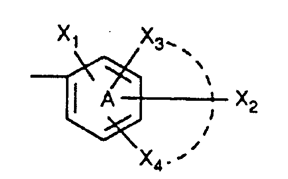
in which X₁ and X₂ independently of one another are hydrogen, chlorine, bromine, NO₂, methyl, methoxy or ethoxy and X₃ and X₄ form a 5- or 6-membered heterocyclic ring which together with A produces a benzimidazolonyl, dihydroxyquinazolinyl, quinolonyl, benzoxazolonyl, phenmorpholonyl, quinazolinonyl or phthalimidyl radical or a radical of the formula 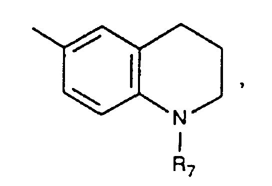 in which R₇ is C₁-C₆alkyl or phenyl, or
Y-R can be a radical

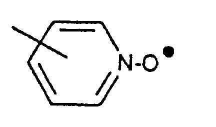

2. A diketopyrrolo[3,4-c]pyrrole according to claim 1 of the formula I in which
X and Y independently of one another are 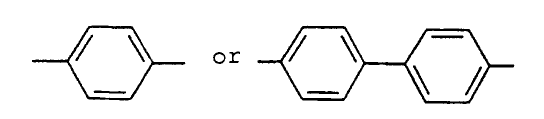
R is a radical CN, NO₂, CO₂R₅ or CON(R₅)₂,
R₁, R₂ and R₅ independently of one another are hydrogen, C₁-C₆alkyl, or phenyl which is unsubstituted or substituted by chlorine, bromine, C₁-C₅alkyl, methoxy, CN or NO₂, or R₁ and R₂, together with the nitrogen atom to which they are attached, form pyrrolidinyl or piperidine.

3. A diketopyrrolo[3,4-c]pyrrole according to claim 1 of the formula I in which X and Y are 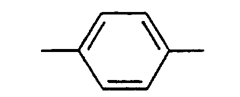
R is a radical CN or NO₂,
R₁ and R₂ independently of one another are hydrogen, methyl, or phenyl which is unsubstituted or substituted by methyl, methoxy, ethoxy, Cl or NO₂, or
R₁ and R₂, together with the nitrogen atom to which they are attached, form pyrrolidinyl or piperidyl.

4. A diketopyrrolo[3,4-c]pyrrole according to claim 3 of the formula I in which R₁ and R₂ are identical.

5. A high molecular weight organic material pigmented with a diketopyrrolo[3,4-c]pyrrole according to claim 1.

## Revendications

1. Dicétopyrrolo[3,4-c]pyrroles de formule 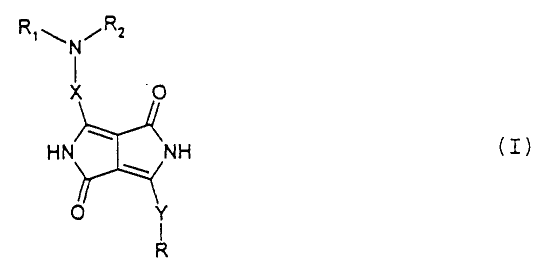 où X et Y représentent, indépendamment l'un de l'autre, un reste aromatique de formule 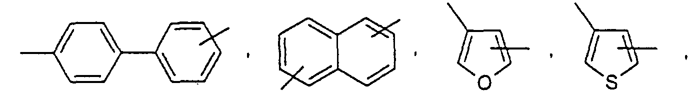 ou 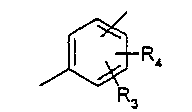
R représente un reste CN, COR₅, CO₂R₅, CON(R₅)₂, NO₂, SO₂R₅, SOR₅, SO₂N(R₅)₂ ou PO(OR₆)₂,
R₃ et R₄ représentent, indépendamment l'un de l'autre, des atomes d'hydrogène, de chlore, de brome, des groupes méthyle, éthyle, méthoxy ou éthoxy,
R₆ représente un groupe alkyle en C₁-C₆ ou phényle,
R₁, R₂ et R₅ représentent, indépendamment, l'un de l'autre, un atome d'hydrogène, des groupes alkyle en C₁-C₁₈, alcényle en C₂-C₁₈, phényle non substitué ou substitué par des substituants chloro, bromo, hydroxy, alkyle en C₁-C₆, alkoxy en C₁-C₆, (alkyl en C₁-C₆)mercapto, CN, NO₂ ou CF₃, ou
R₁ et R₂ forment, ensemble avec l'atome de N auquel ils sont liés, un reste hétérocyclique non substitué ou substitué par un substituant alkyle en C₁-C₆ ou phényle, pris dans le groupe des restes pyrrolidinyle, pipéridyle, pyrrolyle, triazolyle, imidazolyle, pyrazolyle, pipérazinyle, morpholinyle, thiomorpholinyle, carbazol-1-yle, indol-1-yle, indazol-1-yle, benzimidazol-1-yle, tétrahydroquinoléin-1-yle et tétrahydroquinoléin-2-yle, ou lorsque
R₁ représente un atome d'hydrogène, R₂ représente un reste de formule 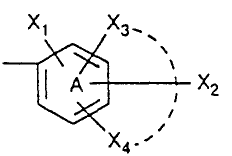
où X₁ et X₂ représentent, indépendamment l'un de l'autre, des atomes d'hydrogène, de chlore, de brome, des groupes NO₂, méthyle, méthoxy ou éthoxy et X₃ et X₄ forment un cycle hétérocyclique qui forment conjointement avec A, un reste benzimidazolonyle, dihydroxyquinazolinyle, quinolonyle, benzoxazolonyle, phénmorpholonyle, quinazolinonyle, phtalimidyle ou un reste de formule 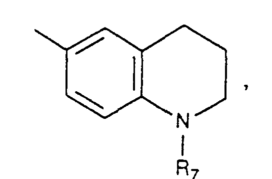 où R₇ représente des groupes alkyle en C₁-C₆ ou phényle, ou
Y-R peut représenter un reste

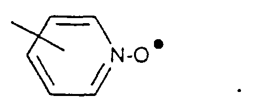

2. Dicétopyrrolo[3,4-c]pyrroles selon la revendication 1, de formule I, où
X et Y représentent, indépendamment l'un de l'autre 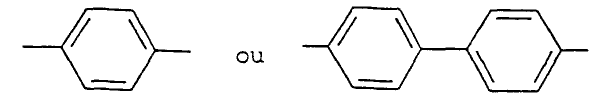
R représente un reste CN, NO₂, CO₂R₅ ou CON(R₅)₂,
R₁, R₂ et R₅ représentent, indépendamment l'un de l'autre, un atome d'hydrogène, un groupe alkyle en C₁-C₆, phényle non substitué ou substitué par des substituants chloro, bromo, alkyle en C₁-C₅, méthoxy, CN ou NO₂, ou R₁ et R₂ forment, ensemble avec l'atome de N auquel ils sont liés, un pyrrolidinyle ou pipéridine.

3. Dicétopyrrolo[3,4-c]pyrroles selon la revendication 1, de formule I, où X et Y représentent un groupe 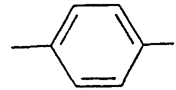
R représente un reste CN ou NO₂,
R₁ et R₂ représentent, indépendamment l'un de l'autre, un atome d'hydrogène, des groupes méthyle, phényle non substitué ou substitué par des substituants méthyle, méthoxy, éthoxy, Cl ou NO₂ ou R₁ et R₂ forment, ensemble avec l'atome de N auquel ils sont liés, un pyrrolidinyle ou pipéridyle.

4. Dicétopyrrolo[3,4-c]pyrroles selon la revendication 3 de formule I, où R₁ et R₂ sont identiques.

5. Matière organique de haut poids moléculaire pigmentée par un dicétopyrrolo-[3,4-c]-pyrrole selon la revendication 1.
